# EUROPEAN PATENT SPECIFICATION

(11) **EP 2 601 963 B1**
(45) Date of publication and mention of the grant of the patent: **22.04.2015**
(21) Application number: 11838304.1
(22) Date of filing: 21.09.2011
(51) Int. Cl.: A61K 38/05, A61P 1/04

(54) **N-ISOVALEROYL-L-GLUTAMYL-L-TRYPTOPHAN AS AN AGENT INHIBITING THE FORMATION OF ULCERS IN THE STOMACH AND THE DUODENUM**
N-ISOVALEROYL-L-GLUTAMYL-L-TRYPTOPHAN ALS MITTEL ZUR HEMMUNG DER BILDUNG VON GESCHWÜREN IM MAGEN UND IM ZWÖLFFINGERDARM
N-ISOVALÉROYL-L-GLUTAMYL-L-TRYPTOPHANE UTILISÉ COMME AGENT EMPÊCHANT LA FORMATION D'ULCÈRES DANS L'ESTOMAC ET LE DUODÉNUM

(30) Priority: 21.10.2010 RU 2010143328
(43) Date of publication of application: 12.06.2013
(73) Proprietor: Obshestvo S Ogranichennoj Otvetstvennostju "CytoNIR", St. Petersburg 191023 (RU)
(72) Inventor: KUDRJAVCEVA, Tatjana Anatoljevna, St.Petersburg 197373 (RU); PETLENKO, Sergej Victorovich, St.Petersburg 195269 (RU); GRIGORJEV, Eugenij losifovich, St.Petersburg 198332 (RU)
(74) Representative: Jeck, Anton
(86) International application number: PCT/RU2011/000720
(87) International publication number: WO 2012/060734

(56) References cited:
- WO-A2-2008/100458
- RU-C1- 2 279 882
- UA-A- 69 730
- US-A- 5 728 680
- KUNIMI HARUYO ET AL.: 'Effects of CL-1700 on duodenal ulcer formation in the rat' JAPAN J. PHARMACOL vol. 32, no. 1, 1982, pages 167 - 70, XP003031458
- MIKIO ITO ET AL.: 'Preventive Actions of N-(3-Aminopropionyl)-L-Histidinato Zinc (Z-103) through Increases in the Activities of Oxygen-Derived Free Radical Scavenging Enzymes in the Gastric Mucosa on Ethanol-Induced Gastric Mucosal Damage in Rats' JAPAN J. PHARMACOL vol. 59, 1992, pages 267 - 274, XP000571038
- FALALYEYEVA T.M. ET AL.: 'Effect of glyprolines on homeostasis of gastric mucosa in rats with stress ulcers' BULL EXP BIOL MED vol. 149, no. I, July 2010, pages 26 - 8, XP055070525

## Description

### Field of the Invention

The invention relates to organic chemistry, specifically to peptide compounds. The claimed compound can be useful for the treatment and prevention of gastric and duodenal ulcers.

### Prior Art

Gastric and duodenal ulcer disease is a chronic periodically recurrent condition and one of the most common gastrointestinal diseases. Today, it is understood that the leading cause of peptic ulcer pathogenesis is the imbalance between the factors attacking the gastric acid and the factors protecting the mucous membranes of the stomach and duodenum. Since peptic ulcers are polygenic, the pharmacotherapy of the disease includes various groups of drugs directed at the main components of the pathogenesis of ulcer formation: antacids, H₂-blockers and proton pump inhibitors, antibiotics, anti-Helicobacter agents, etc. [Yu.V. Vasilyev, Coating (antacid) Medications for the Treatment of Some Upper Digestive Diseases. // Gastroenterology, Addendum to Consillum Medicum, 2004, #5, pp. 244 - 248; Yu.V. Vasilyev, Contemporary Therapy of Peptic Ulcer Disease Associated with Helicobacter Pilori. // Trudny Patsient, 2007, #6 - 7, p.p. 35 - 41; Yu.V. Vasilyev, Peptic Ulcer Disease// Selected Chapters of Clinical Gastroenterology / edited by L.B. Lazebnik, M: Anakharsis, 2005, p.p. 82 - 112].

Presently, there are nearly 450 known antiulcer medications with different mechanisms of action. However, there is not even one medication that doesn't cause any adverse side effects, especially after long-term use. Therefore, it is very important to find a preventive treatment for gastric and duodenal ulcers.

### Disclosure of the Invention

The objective of the invention is to synthesize a novel compound of the peptide class, which would prevent the formation of gastric and duodenal ulcers triggered by medications or stressors or accompanying diseases of other internal organs.

The stated objective is achieved with the synthesis of N-isovaleroyl-L-glutamyl-L-tryptophan with the following formula: 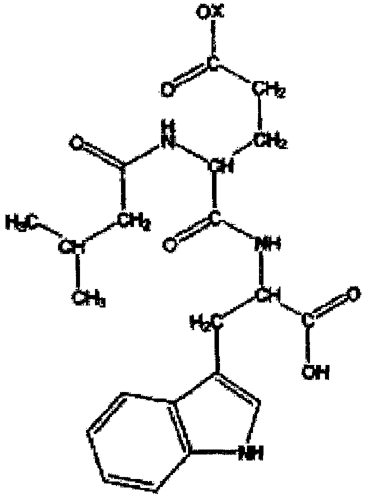 where X = H, Na, K, with the 417.5 - 456.5 molecular weight, as an agent preventing the formation of gastric and duodenal ulcers.

The claimed chemical compound was first synthesized in our laboratory and has no structural analogues (N-protected peptides) known from prior art.

Synthesis of N-isovaleroyl-L-glutamyl-L-tryptophan (C₂₁H₂₇N₃O₆) was carried out in two stages:

### 1. Preparation of isovaleric acid N-oxysuccinimide ester.

4.0 g of N-oxysuccinimide were added to 3.0 ml (30 mM) of isovaleric acid dissolved in 30 ml of tetrahydrofuran and the resulting solution was cooled in an ice bath. 7.2 (35 mM) of N,N'-dicyclohexylcarbodiimide were dissolved in 30 ml of tetrahydrofuran and cooled in a similar manner. The resulting solutions were combined and stirred while cooling in ice for 3 hrs., and then for two days at room temperature.

The precipitated N,N'-dicyclohexylurea was filtered, the solvent evaporated in a rotary vacuum evaporator at 40°C, the residue dissolved in a minimum amount of ethyl acetate and crystallized by adding hexane. The resulting residue was filtered and recrystallized from isopropyl alcohol. The final product was dried in vacuum over KOH and P₂O₅. Yield: 4.7 g (76%).

The product was found to be homogeneous according to the data obtained by thin-layer chromatography: mobility R_{f} 0.67 (benzene-acetone system 1:1, Silufol, chlorine/benzidine development).

### 2. Preparation of N-isovaleroyl-L-glutamyl-L-tryptophan

1.0 g (3 mM) glutamyl-tryptophan monosodium salt was added to 1.2 g (6 mM) of isovaleric acid N-oxysuccinimide ester suspended in 30 ml of the dioxane -water mixture (1 : 1)with vigorous stirring. The stirring continued for two days. pH of the reaction mixture was kept at the 8.0 - 8.5 range with the addition of 0.1 N NaOH solution. Upon completion of the reaction, the mixture was brought to pH = 3 with 0.5 N Na₂SO₄ solution and extracted with ethyl acetate (3 x 50 ml). The combined organic layer was successively washed with 0.5 N H₂SO₄ solution (3 x 50 ml), water (2 x 50 ml), 2.5 % NaHCO₃ solution (1 x 50 ml) cooled to 0°C, water (2 x 50 ml), 0.5 N H₂SO₄ solution (2 x 50 ml), water (2 x 50 ml) and saturated Na₂SO₄solution (2 x 50 ml). The organic layer was dried over anhydrous Na₂SO₄, the solvent evaporated in a rotary vacuum evaporator at 40°C, and the residue crystallized in hexane. The resulting precipitate was filtered and dried in vacuum over KOH and P₂O₅. Yield 1.1 g (80 %).

The product was found to be homogeneous according to the data obtained by thin-layer chromatography: mobility R_{f} 0.27 (benzene - acetone system 1 : 1, Silufol plate, chlorine/benzidine development).

The content of the main substance was determined by high-performance liquid chromatography (HPLC) on a Beckman chromatograph, Supelco LC-18-DB column, 4.6 x 250 mm. Mobile phase A: 0.1 % TFA (trifluoroacetic acid), mobile phase B: MeCN (acetonitrile) /0.1 % TFA; gradient B: 0 -> 20% in 30 min. Flow rate 1 ml/min. Detection at 220 nm, scanning at 190 - 600 nm, sample - 20 mkL. The content of the main substance is 98.45 %.

Calculated % for C₂₁H₂₇N₃O₆: C (60.42), H (6.52), N (10.07); actual % C (60.31), H (6.63), N (9.95); PMR-spectrum (proton magnetic resonance) was recorded on a Bruker AM spectrophotometer, 300 MHz in DMSO (dimethylsulfoxide): 6.97 (CH₃)_{;} 1.71 (CH); 1.97 (CH₂); 3.0 (CH); 4.92 (CH₂); 7.20, 7.08, 6.92, 6.82 (CH indole): 7.47 (NH indole); 7.84, 7.94 (NH amide); 10.63 (COOH). UV-spectrum (absorption spectrum in the UV-region) was recorded on a Beckman spectrophotometer: λₘₐₓ 280 nm, shoulder 286 nm (0.004 % solution in 0.01 M NaOH).

### 3. Preparation of N-isovaleroyl-L-glutamyl-L-tryptophan Sodium Salt

0.01 N solution of NAOH was added to 600 mg of N-isovaleroyl-L-glutamyl-L-tryptophan suspended in 50 ml of distilled water to render pH = 7. The resulting solution was evaporated in a rotary vacuum evaporator, the reaction product was crystallized from a minimum amount of water with isopropyl alcohol. The precipitate was filtered and dried in vacuum over KOH and P₂O₅.

The potassium salt was similarly prepared.

A general toxicology study of the claimed composition was carried out.

Total toxicity of N-isovaleroyl-L-glutamyl-L-tryptophan was studied in accordance with the requirements of The Handbook of the Experimental (non-clinical) Studies of Novel Pharmaceutical Substances (2000): single dose acute toxicity and sub-acute and chronic toxicity during long-term administration of the dipeptide.

Acute toxicity testing was conducted on 60 white outbred male mice with 20 - 23 g body weights. The animals were divided into 6 equal groups. Single intramuscular doses of the medication were administered to the animals in the amount of 0.1 ml in the following doses: 1 mg/kg, 5 mg/kg, 10 mg/kg, and 20 mg/kg in sterile 0.9 % NaCl solution. The animals in the control group were receiving the same amounts of 0.9 % NaCl solution.

Sub-acute toxicity testing was conducted on 60 white outbred male mice weighing 150 - 250 g. Single daily doses of the medication were administered to the animals in the experimental groups over 90 days in the following doses: 100 mkg/kg, 1 mg/kg, and 10 mg/kg in 0.25 ml of 0.9 % NaCl. The animals in the control group were receiving the same amounts of 0.9 % NaCl solution. The morphological composition and the properties of the peripheral blood of the animals were examined prior to the administration of the preparation and on the 30, 60, and 90 day after administration. The biochemical and coagulation properties of the blood were studied upon completion of the experiment.

Chronic toxicity study was conducted over 6 months on 100 male guinea pigs weighing 300 - 350 g; the length of the study was determined by the recommended course of treatment with the preparation. Daily single intramuscular doses of the medication were administered to the animals in the experimental groups over 6 months in the following doses: 100 mkg/kg, 1 mg/kg, and 10 mg/kg in 0.5 ml of 0.9 % NaCl. The animals in the control group were receiving the same amounts of 0.9 % NaCl solution in the same regimen.

Peripheral blood of the animals was analyzed by conventional procedures for erythrocytes, hemoglobin, reticulocytes, thrombocytes, leukocytes, differential white blood cell count, erythrocyte sedimentation rate (ESR), and blood fragility. In addition, the total protein in blood serum was analyzed by Lowry protein assay and potassium and sodium were analyzed by plasma spectrophotometry. Upon completion of the experiment, a pathomorphological study was performed on the brain, spinal fluid, spinal ganglions, thyroid gland, parathyroid gland, adrenal glands, testicles, pituitary gland, heart, lungs, aorta, liver, kidneys, bladder, pancreas, stomach, small intestine, large intestine, thymus, lymph nodes, and bone marrow.

The acute toxicity study showed that a single-dose administration of the dipeptide to the animals in a dose exceeding the recommended therapeutic dose for clinical use more than 100 times did not cause any toxic reactions, which shows that the therapeutic index of the drug is very high.

As N-isovaleroyl-L-glutamyl-L-tryptophan is nontoxic, it may be recommended for clinical studies of experimental ulcer disease in vivo.

A pharmacological effect of the claimed compound was studied on the experimental models of gastric and duodenal ulcers. Notably, trophic disorders of mucous membranes and the reduced overall energy and plasticity maintenance of the gastrointestinal tissues play an important role in the pathogenesis of gastrointestinal lesions induced by nonsteroidal anti-inflammatory drugs (NSAIDs) or by stressors. Thus, the ulcerogenic effect is not only directed at the gastric mucosa, but also at the duodenum.

Three series of experiments were conducted:
- A study of the preventive antiulcer effect of N-isovaleroyl-L-glutamyl-L-tryptophan (VGT) on the water immersion stress model.
- A study of the preventive effect of the course of VGT administration on the formation of indomethacin-induced gastric ulcers.
- A study of the therapeutic effect of VGT on the indomethacin-induced ulcer model.

All studies were conducted on male Wistar rats weighing 220 ± 20 g, obtained from Rappolovo nursery (Leningrad oblast). For 14 days prior to the experiment, the animals were held in isolation. The animals were handled in accordance with the European Convention for the Protection of Vertebrate Animals used for Experimental and Other Scientific Purposes (Strasburg, 1986). The animals were held in standard plastic cages and fed a standard diet of PK120-3 (granulated feed in accordance with order # 1179 M3 USSR 10/10/1983) with a free access to water.

In each series, the animals were divided into experimental and control groups with 10 rats per group. The animals in the experimental groups were orally administered the claimed preparation or a comparator drug, and the control group animals received distilled water in the same amount.

### Example 1: Efficacy of preventive treatment with N-isovaleroyi-L-glutamyl-L-tryptophan in water-immersion stress model.

The experimental animals were divided into 5 groups, 10 animals each. The animals received the following therapy:
- control group received distilled water per os;
- experimental group 1 received 0.1 mkg/kg N-isovaleroyl-L-glutamyl-L-tryptophan;
- experimental group 2 received 10 mkg/kg N-isovaleroyl-L-glutamyl-L-tryptophan;
- experimental group 3 received 200 mkg/kg of the comparator drug Sucralfate;
- experimental group 4 received 50 mkg/kg of the comparator drug Misoprostol;

Sucralfate contains an aluminum complex of beta-D-fructofuranosyl-alpha-D-glucopyranoside octakis (hydrosulfate) as an active ingredient. This drug is recommended for the treatment and short-term prevention of gastropathy induced by non-steroidal anti-inflammatory drugs (NSAIDs) to be administered 3 - 4 times a day. As with other aluminum-containing medication, it is not suitable for long-term administration.

Misoprostol - (11 alpha, 13E)-(+-)-11,16-dihydroxy-16-methyl-9-oxaprost-13-en-1-ic acid methyl ester is approved for the prevention of NSAID-induced gastropathy.

The experiment was conducted as follows:
The proposed drug, comparator drugs, and distilled water were administered once daily over 7 days, and on the day of the ulcerogenic stress induction one hour prior to the stress.

18 hrs. prior to the ulcerogenic stress induction, the animals were not fed but had a free access to water.

The rats deprived of food for 18 hrs. were placed into special cases and immersed in water vertically to the level of their xyphoids. The animals were held in this position for 5 hrs. at 23° C water temperature. After that, the animals were removed from the experiment.

Animals' body weight changes were monitored throughout the experiment. The experimental animals were then sacrificed by decapitation, and their blood was collected in test tubes. The stomachs were extracted, dissected along the major curve, rinsed with cold saline solution, and the mucosa was carefully examined through a magnifying glass under bright lights. The mucosa was inspected for color, the presence of hyperemia and its intensity. The number of lesions, which were differentiated into small round (less than 1 mm), linear, and large lesions (more than 1mm) and the size of the area they covered were calculated. The average number of lesions per one animal in a group and the percentage of animals with ulcers were also calculated. Pauls Index (PI) was used as an integral indicator of the number of lesions and was calculated as follows:
PI = average number of lesions in a group x % of animals with lesions/100 [Pauls F., Wick A.M.,Mac. Key E.M. et al. An assau method for anti ulcer substances // Gastroenterology.- 1947. #8.- p.p.774 - 782]. Ulcer index UI was calculated as: UI = average lesion area size in the group x % of animals with lesions/100; gastroprotective activity GA₁ was calculated as: GA₁ = PI of the control group/PI of the experimental group, GA₂ = UI of the control group/UI of the experimental group. The proposed drug was considered active when GA ≥ 2.

Intensity of the free radical oxidation was evaluated by the malondialdehyde (MDA) accumulation in blood serum [R.A. Temirbulatov, Ye.I. Seleznev Method for Increasing the Intensity of Free Radical Oxidation in Lipid-Containing Blood Components and its Diagnostic Significance // Labor. Delo, 1981, # 4, p.p. 209 - 211]. The state of the antioxidant systems was evaluated by the superoxide dismutase (SODM) activity [S. Chevari , I. Chaba, Y. Sekei. The Role of Superoxide Dismutase in Cellular Oxidative Processes and Method of its Determination in Biological Materials // Labor. Delo, 1985, # 11, p.p. 678 - 681].

Alanine aminotransferase (ALT) and alkaline phosphatase (AP) activity in blood serum was determined with "Vital Development Corporation" kits.

Intensity of changes caused by stressors in the internal organs of the animals was evaluated by the relative weight changes of the adrenal glands, thymus, and spleen calculated as a ratio of the organ's weight to the body weight of the animal multiplied by 100.

Statistical analysis was conducted with a STATISTICA 6 package of statistical software.

Animals are known to respond to stressors with a complex of changes to the internal organs [H. Selye Stress Adaptation Syndrome, M., Medgiz, 1960, p.207] known as the "stress triad," including adrenal hypertrophy, immunocompetent organ involution, and lesions in the gastric mucosa.

The experiment demonstrated that the animals under a 5-hour water-immersion stress developed pronounced hyperemia of gastric mucosa and also formed ulcers and erosions. Additionally noted were adrenal hypertrophy and increased lipid peroxidation LP (Tables 1 - 4).

**Table 1**

| ***Effect of Preventive Treatment with N-isovaleroyl-L-glutamyl-L-tryptophan on the Formation of Stress-Induced Ulcers (M ± SEM)*** | | | | | | | | |
|---|---|---|---|---|---|---|---|---|
| Groups | Large round | | Linear | | Small round | | Total | |
| | Amt. | Size | Amt. | Size | Amt. | Size | Amt. | Size |
| Control stress | 12.5 ± 0.8 | 11.8 ± 0.7 | 2.4 ± 0.3 | 5.6 ± 1.8 | 7.2 ± 0.4 | 2.9 ± 0.3 | 22.1 ± 1.1 | 20.2 ± 1.6 |
| VGT preparation 0.1 mkg/kg + stress | 6.9 ± 1.4* | 4.4 ± 1.1* | 1.5 ± 0.7 | 2.4 ± 1.0 | 7.0 ± 1.4 | 1.9 ± 0.4 | 15.4 ± 2.1 | 8.7 ± 2.0* |
| VGT preparation 10.0 mkg/kg + stress | 5.7 ± 1.1* | 3.4 ± 0.7* | 1.4 ± 0.3 | 3.9 ± 1.4 | 9.2 ± 1.5 | 2.5 ± 0.4 | 16.3 ± 1.5 | 9.7 ± 1.2* |
| Sucralfate 200 mg/kg + stress | 3.9 ± 0.8* | 2.4 ± 0.6* | 2.5 ± 0.6 | 4.5 ± 1.0 | 2.4 ± 0.7 | 0.7 ± 0.2 | 8.8 ± 1.4* | 7.5 ± 1.5* |
| Misoprostol 50 mkg/kg + stress | 5.2 ± 1.1* | 3.1 ± 0.7* | 1.8 ± 0.5 | 2.6 ± 0.7 | 6.6 ± 1.3 | 1.7 ± 0.3 | 13.6 ± 2.4* | 7.3 ± 1.3* |

| | | | | | | | | |
|---|---|---|---|---|---|---|---|---|
| * Significant difference compared to control (stress) group, P < 0.05 | | | | | | | | |

Preventive intraperitoneal administration of the preparation in both doses reduced the number and size of stress-induced gastric ulcers. The effect of N-isovaleroyl-L-glutamyl-L-tryptophan was dose-dependent and this relation was inverse. A reduction in the size of the lesions was more pronounced since the change in the number of ulcers per animal in a group occurred mostly on the account of the smaller number of large lesions (Table 1). Thus, VGT preparation reduced the size of large lesions in 2.7 and 3.4 times depending on the dose. The preventive effect of the smaller dose was more pronounced in the prevention of the formation of the total number of lesions and their reduced size. Additionally, preventive treatment with N-isovaleroyl-L-glutamyl-L-tryptophan in a 0.1 mkg/kg dose was shown to reduce the number of animals with ulcers per group comparable to Sucralfate. Calculated ulcer and gastroprotective activity indices revealed that VGT in a 0.1 mkg/kg dose showed antiulcer activity comparable to that of Sucralfate or Misoprostol. Thus, the size of lesions in the animas receiving N-isovaleroyl-L-glutamyl-L-tryptophan was 2.1 - 2.6 times smaller than in the control group animals.

**Table 2**

| ***Gastroprotective Activity (GA) of N-isovaleroyl-L-glutamyl-L-tryptophan during Water-Immersion-Induced Stress (M* ± *SEM)*** | | | | | | | |
|---|---|---|---|---|---|---|---|
| Groups | # Lesions | Lesion Size | % Animals with Ulcers | Pauls Index | Ulcer Index | GA₁ | GA₂ |
| Control stress | 22.1 ± 1.1 | 20.2 ± 1.6 | 100 | 22.1 | 20.2 | | |
| VGT preparation 0.1 mkg/kg + stress | 15.4 ± 2.1 | 8.7 ± 2.0* | 90 | 13.9 | 7.83 | 1.59 | 2.58 |
| VGT preparation 10 mkg/kg + stress | 16.3 ± 1.5 | 9.7 ± 1.2* | 100 | 16.3 | 9.7 | 1.36 | 2.08 |
| Sucralfate 200 mg/kg + stress | 8.8 ± 1.4* | 7.5 ± 1.5* | 90 | 7.92 | 6.75 | 2.79 | 2.99 |
| Misoprostol 50 mkg/kg + stress | 13.6 ± 2.4* | 7.3 ± 1.3* | 100 | 13.6 | 7.3 | 1.63 | 2.77 |

| | | | | | | | |
|---|---|---|---|---|---|---|---|
| * - Significant difference compared to control (stress) group, P < 0.05 GA₁₋ gastroprotective activity calculated for number of lesions GA₂ - gastroprotective activity calculated for lesion size | | | | | | | |

**Table 3**

| ***Superoxide Dismutase Activity and Malondialdehyde Content in Blood Serum of Rats Subjected to Water-Immersion-Induced Stress (M* ± *SEM)*** | | |
|---|---|---|
| Groups | SODM (con. units/ml) | MDA (OD₅₃₂) |
| Intact Animals | 2.20 ± 0.17 | 0.103 ± 0.016 |
| Control stress | 0.96 ± 0.09# | 0.271 ± 0.020# |
| VGT Preparation 0.1 mkg/kg + stress | 2.20 ± 0.09* | 0.130 ± 0.019* |
| VGT Preparation 10 mkg/kg + stress | 2.35 ± 0.11* | 0.128 ± 0.020* |
| Sucralfate 200 mg/kg + stress | 2.32 ± 0.11* | 0.147 ± 0.007* |
| Misoprostol 50 mkg/kg + stress | 2.58 ± 0.17* | 0.151 ± 0.11* |

| | | |
|---|---|---|
| # - Significant difference compared to intact animals, P < 0.05 * - Significant difference compared to control (stress) group, P < 0.05 | | |

Thus, these data confirm that the proposed N-isovaleroyl-L-glutamyl-L-tryptophan preparation exhibits a preventive effect, raising resistance of gastric mucosa to the damaging effect from the stress.

Gastric mucosa also showed increased resistance to excessive lipid peroxidation (LP) and maintained enzymatic activity of the antioxidant system (AOS) in blood serum of rats (Table 3). N-isovaleroyl-L-glutamyl-L-tryptophan in the proposed doses prevented reduction of SODM activity and elevated MDA production.

**Table 4**

| ***Effect of N-isovaleroyl-L-glutamyl-L-tryptophan on the Activity of Alanine Aminotransferase and Alkaline Phosphatase in Blood Serum of Rats Subjected to Water-Immersion-Induced Stress (M* ± *SEM)*** | | |
|---|---|---|
| Groups | ALT (mM/hr-L) | AP (nM/s-L) |
| Intact Animals | 1.08 ± 0.03 | 1037 ± 65 |
| Control stress | 2.20 ± 0.23# | 1496 ± 36# |
| VGT Preparation 0.1 mkg/kg + stress | 1.55 ± 0.15* | 1100 ± 54* |
| VGT Preparation 10.0 mkg/kg + stress | 1.78 ± 0.08* | 1123 ± 55* |
| Sucralfate 200 mg/kg + stress | 1.57 ± 0.06* | 1113 ± 77* |
| Misoprostol 50 mkg/kg + stress | 1.60 ± 0.06* | 976 ± 76* |

| | | |
|---|---|---|
| # - Significant difference compared to intact animals, P < 0.05 *- Significant difference compared to control (stress) group, P < 0.05 | | |

Preventive administration of the preparation helped maintain the alanine aminotransferase (ALT) and alkaline phosphatase (AF) activity at a level close to that of the intact animals, notably, the VGT effect was more pronounced at the 0.1 mkg/kg dose (Table 4).

Calculated relative weight of the spleen and thymus demonstrated that no significant changes to these organs occurred in animals under stress, while relative weight of the adrenal glands increased significantly, by 18 % (Table 5). N-isovaleroyl-L-glutamyl-L-tryptophan in the 0.1 mkg/kg dose prevented adrenal hypertrophy by reducing their weight by 16 - 17 % compared to that of the control animals. Administration of small doses of the preparation lowered the relative weight of the spleen by 24 % compared to that of the intact animals. These data, therefore, demonstrated that VGT offered protection from stress by reducing the damage to gastric mucosa, preventing adrenal hypertrophy, excessive lipid peroxidation, and impairment of the SODM protective function and increasing the activity of tissue damage marker enzymes.

**Table 5**

| ***Effect of Preventive Treatment with N-isovaleroyl-L-glutamyl-L-tryptophan on the Weight of Organs of Rats Subjected to Stress (M* ± *SEM)*** | | | | | | | |
|---|---|---|---|---|---|---|---|
| Groups | Animal | Spleen | | Adrenal Glands | | Thymus | |
| | Wt. (g) | Wt., g | Rel | Wt., g | Rel | Wt., g | Rel |
| | | Wt., % | | Wt., % | | Wt., % | |
| Intact Animals | 232 ± 6 | 1.148 ± 0.0999 | 0.49 ± 0.048 | 41.7 ± 1.8 | 0.017 ± 0.001 | 0.290 ± 0.017 | 0.125 ± 0.007 |
| Control stress | 241 ± 7 | 1.0 ± 0.059 | 0.42 ± 0.07 | 48.3 ± 2.6# | 0.020 ± 0.001# | 0.37 ± 0.025 | 0.145± 0.01 |
| VGT Preparation 0.1 mkg/kg + stress | 236± 7 | 0.872 ± 0.066 | 0.37 ± 0.06* | 40.8 ± 1.1* | 0.017 ± 0.001 * | 0.307 ± 0.028 | 0.124 ± 0.010* |
| VGT Preparation 10.0 mkg/kg + stress | 239 ± 6 | 0.977 ± 0.082 | 0.41 ± 0.03 | 45.2 ± 2.2 | 0.018 ± 0.001* | 0.350 ± 0.022 | 0.154 ± 0.008 |
| Sucralfate 200 mg/kg + stress | 240 ± 7 | 0.974 ± 0.057 | 0.40 ± 0.02 | 41.6 ± 2.7 | 0.017 ± 0.001* | 0.327 ± 0.016 | 0.140 ± 0.007 |
| Misoprostol 50 mkg/kg + stress | 229 ± 6 | 0.886 ± 0.052 | 0.39 ± 0.02 | 41.0 ± 0.8* | 0.018 ± 0.001 | 0.323 ± 0.030 | 0.140 ± 0.011 |

| | | | | | | | |
|---|---|---|---|---|---|---|---|
| # - Significant difference compared to intact animals, P < 0.05 *- Significant difference compared to control group, P < 0.05 | | | | | | | |

### Example 2: Efficacy of preventive treatment with N-isovaleroyl-L-glutamyl-L-tryptophan in oral indomethacin-induced ulcer model.

Five groups of 10 animals per group were prepared for the experiment as described in *Example 1.*

In each series of experiments, the animals were divided into experimental and control groups at 10 animals per group. The animals in the experimental group were orally administered the proposed medication or a comparator drug, and the control animals were given distilled water in the same amount.
- control group received distilled water per os;
- experimental group 1 received 0.1 mkg/kg N-isovaleroyl-L-glutamyl-L-tryptophan;
- experimental group 2 received 10 mkg/kg N-isovaleroyl-L-glutamyl-L-tryptophan;
- experimental group 3 received 200 mkg/kg of the comparator drug Sucralfate.

Indomethacin is a non-steroidal anti-inflammatory drug that has an ulcerogenic effect on the gastric mucosa.

The efficacy of the course of preventive treatment with the claimed composition was evaluated 24 hrs. post indomethacin administration. The study was conducted as in *Example 1.*

Indomethacin administered in ulcerogenic doses was shown to cause destruction of the gastric mucosa, mostly producing large or linear lesions. Ulcers of gastric mucosa were accompanied by reduced SODM activity and increased amount of MDA, elevated ALT and AP activity in the blood serum, and changes to the relative weight of the organs (significantly increased relative weight of the liver and spleen and strong tendency for this indicator to increase in the adrenal glands and thymus) (Table 6 - 9).

Preventive treatment with VGT significantly inhibited the formation of indomethacin-induced gastric ulcer lesions caused by indomethacin (Table 6). The size of the lesions in the gastric mucosa of the animals receiving VGT was reduced 5 times.

**Table 6**

| ***Effect of Preventive Treatment with N-isovaleroyl-L-glutamyl-L-tryptophan on the Formation of Indomethacin-Induced Ulcers (M* ± *SEM)*** | | | | |
|---|---|---|---|---|
| Groups | Large round | | Linear | |
| | Amount | Size | Amount | Size |
| Indomethacin control | 7.9 ± 1.0 | 5.2 ± 0.7 | 6.9 ± 1.0 | 15.2 ± 4.0 |
| VGT Preparation 0.1 mkg/kg | 2.7 ± 0.5* | 1.4 ± 0.3* | 2.0 ± 0.7* | 1.9 ± 0.7* |
| VGT Preparation 10.0 mkg/kg | 3.5 ± 0.9 | 1.7 ± 0.6* | 1.2 ± 0.5* | 1.8 ± 1.1* |
| Sucralfate 200 mg/kg | 2.4 ± 0.8* | 1.0 ± 0.4* | 1.2 ± 0.4* | 1.8 ± 0.9* |
| | | | | |

| Groups | Small round | | Total | |
|---|---|---|---|---|
| | Amount | Size | Amount | Size |
| Indomethacin control | 6.5 ± 1.3 | 2.2 ±0.5 | 21.3 ± 2.1 | 22.6 ± 4.5 |
| VGT Preparation 0.1 mkg/kg | 2.8 ± 0.7 | 0.8 ± 0.2 | 7.5 ± 1.0* | 4.1 ± 0.9* |
| VGT Preparation 10.0 mkg/kg | 3.6 ± 1.3 | 0.5 ± 0.1 | 8.3 ± 1.7* | 4.1 ± 1.6* |
| Sucralfate 200 mg/kg | 1.8 ± 0.6 | 0.5 ± 0.2* | 5.4 ± 1.4* | 3.3 ± 1.2* |

| | | | | |
|---|---|---|---|---|
| *- Significant difference compared to control group, P < 0.05 | | | | |

Calculated and compared integral antiulcer activity indicators revealed that isovaleroyl glutamyl tryptophan has a pronounced gastroprotective effect (Table 7)

**Table 7**

| ***Gastroprotective Activity of N-isovaleroyl-L-glutamyl-L-tryptophan in Modulated Indomethacin-Induced Gastric Ulcers (M* ± *SEM)*** | | | | | | | |
|---|---|---|---|---|---|---|---|
| Groups | # Lesions | Lesion Size | % Animals with Ulcers | PI | Ulcer Index | GA₁ | GA₂ |
| Control Indomethacin 20 mg/kg | 21.3 ± 2.1 | 22.6 ± 4.5 | 100 | 21.3 | 22.6 | | |
| VGT preparation 0.1 mkg/kg + Indomethacin | 7.5 ± 1.0* | 4.1 ± 0.9* | 100 | 7.5 | 4.1 | 2.84 | 5.51 |
| VGT preparation 10 mkg/kg + Indomethacin | 8.3 ± 1.7* | 4.1 ± 1.6* | 100 | 8.3 | 4.1 | 2.57 | 5.51 |
| Sucralfate 200 mg/kg + stress | 5.4 ± 1.4* | 3.3 ± 1.2* | 80 | 4.32 | 2.64 | 4.93 | 8.56 |

| | | | | | | | |
|---|---|---|---|---|---|---|---|
| * - Significant difference compared to control group, P < 0.05 GA₁₋ gastroprotective activity calculated for number of lesions GA₂ - gastroprotective activity calculated for lesion size | | | | | | | |

In addition, indomethacin in the 20 mg/kg dose was found to induce a pronounced free radical oxidation and a concurrent impaired antioxidant state of the body (Table 8). Preventive administration of the claimed preparation reduced the final LP-MDA product concentration by 14 % and increased the activity of the endogenic antioxidant system enzyme SODM 1.6 times compared to similar indicators of the control animals.

**Table 8**

| ***Effect of Preventive Administration of N-isovaleroyl-L-glutamyl-L-tryptophan on the Activity of Alanine Aminotransferase and Alkaline Phosphatase in Blood Serum of Rats in Indomethacin-Induced Ulcer Formation (M* ± *SEM)*** | | |
|---|---|---|
| Groups | SODM (con. units/ml) | MDA (OD₅₃₂) |
| Intact Animals | 2.54 ± 0.13 | 0.103 ± 0.016 |
| Control Indomethacin 20 mg/kg | 1.52 ± 0.12# | 0.268 ± 0.020# |
| VGT Preparation 0.1 mkg/kg + Indomethacin | 2.50 ± 0.05* | 0.124 ± 0.009* |
| VGT Preparation 10 mkg/kg + Indomethacin | 2.58 ± 0.14* | 0.158 ± 0.030* |
| Sucralfate 200 mg/kg + Indomethacin | 2.68 ± 0.14* | 0.134 ± 0.024* |

| | | |
|---|---|---|
| # - Significant difference compared to intact animals, P < 0.05 *- Significant difference compared to control group, P < 0.05 | | |

**Table 9**

| ***Effect of N-isovaleroyl-L-glutamyl-L-tryptophan on the Activity of Alanine Aminotransferase and Alkaline Phosphatase in Blood Serum of Rats in Indomethacin-Induced Ulcer Formation (M* ± *SEM)*** | | |
|---|---|---|
| Groups | ALT (mM/hr-L) | AP (nM/s-L) |
| Intact Animals | 1.03 ± 0.10 | 1037 ± 65 |
| Control 20 mg/kg Indomethacin | 1.42 ± 0.07# | 1277 ± 84# |
| VGT Preparation 0.1 mkg/kg + Indomethacin | 1.04 ± 0.06* | 1108 ± 91 |
| VGT Preparation 10 mkg/kg + Indomethacin | 1.08 ± 0.06* | 1180 ± 91 |
| Sucralfate 200 mg/kg + Indomethacin | 1.05 ± 0.05* | 931 ± 108 |

| | | |
|---|---|---|
| # - Significant difference compared to intact animals, P < 0.05 *- Significant difference compared to control group, P < 0.05 | | |

N-isovaleroyl-L-glutamyl-L-tryptophan administration in both doses impeded an increase in the ALT indomethacin-induced activity in blood serum. The claimed preparation had no significant effect on the activity of alkaline phosphatase (Table 9).

In addition, administration of VGT in the proposed doses maintained relative weights of the spleen, adrenal glands, and thymus at the levels characteristic of those in the intact animals (Table 10).

**Table 10**

| ***Effect of Preventive Treatment with N-isovaleroyl-L-glutamyl-L-tryptophan on the Weight of Organs of Rats in Indomethacin-Induced Ulcer Formation (M* ± *SEM)*** | | | | | |
|---|---|---|---|---|---|
| Groups | Wt. of the Animals (g) | Liver | | Spleen | |
| | | Wt., g | Rel. wt., % | Wt., g | Rel. wt., % |
| Intact Animals | 232 ± 18 | 6.679 ± 0.189 | 2.945 ± 0.057 | 1.021 ± 0.064 | 0.447 ± 0.026 |
| Control 20 mg/kg Indomethacin | 239 ± 12 | 7.894 ± 0.327 | 3.230 ± 0.045# | 1.368 ± 0.076# | 0.542 ± 0.031# |
| VGT Preparation 0.1 mkg/kg + Indomethacin | 219 ± 7 | 6.549 ± 0.282* | 2.99 ± 0.060 | 0.912 ± 0.044* | 0.433 ± 0.041 |
| VGT Preparation 10 mkg/kg + Indomethacin | 216 ± 7 | 6.770 ± 0.335* | 3.053 ± 0.065 | 0.926 ± 0.060* | 0.434 ± 0.018 |
| Sucralfate 200 mg/kg + Indomethacin | 235 ± 9 | 7.702 ± 0.224 | 3.259± 0.049 | 1.070± 0.041 | 0.490 ± 0.020 |

| | | | | | |
|---|---|---|---|---|---|
| | | | | | |

| Groups | Wt. of the Animals (g) | Adrenal Glands | | Thymus | |
|---|---|---|---|---|---|
| | | Wt., g | Rel. wt., % | Wt., g | Rel. wt., % |
| Intact Animals | 232 ± 18 | 40.4 ± 1.4 | 0.018 ± 0.001 | 0.290 ± 0.017 | 0.125 ± 0.007 |
| Control 20 mg/kg Indomethacin | 239 ± 12 | 44.9 ± 2.1 | 0.020 ± 0.001 | 0.261 ± 0.0.039 | 0.163 ± 0.013 |
| VGT Preparation 0.1 mkg/kg + Indomethacin | 219± 7 | 37.1 ± 1.3* | 0.017 ± 0.001 | 0.277 ± 0.018 | 0.124 ± 0.007* |
| VGT Preparation 10 mkg/kg + Indomethacin | 216 ± 7 | 38.5 ± 1.4 | 0.018 ± 0.001 | 0.320 ± 0.030 | 0.133 ± 0.009 |
| Sucralfate 200 mg/kg + Indomethacin | 235±9 | 417 ± 1.5 | 0.017 ± 0.001 | 0.306 ± 0.023 | 0.130 ± 0.009 |

| | | | | | |
|---|---|---|---|---|---|
| # - Significant difference compared to intact animals, P < 0.05 *- Significant difference compared to control group, P < 0.05 | | | | | |

The shown results demonstrated that a course of preventive treatment with the claimed compound exhibits a gastroprotective effect on the indomethacin-induced damage model.

### Example 3: Efficacy of treatment with N-isovaleroyl-L-glutamyl-L-tryptophan in indomethacin-induced ulcer model.

Five groups of 10 animals per group were prepared for the experiment as described in *Example 1.*

In each series of the experiments, the animals were divided into experimental and control groups at 10 animals per group. The proposed medication or a comparator drug were orally administered to the animals in the experimental group, and the control animals were given distilled water in the same amount.
- control group received distilled water per os;
- experimental group 1 received 0.1 mkg/kg isovaleroyl glutamyl tryptophan;
- experimental group 2 received 10 mkg/kg isovaleroyl glutamyl tryptophan;
- experimental group 3 received 200 mkg/kg of the comparator drug Sucralfate.

2 ml/kg of the ulcerogenic dose of indomethacin equal to 30 mg/kg was administered intraperitoneally. The treatment started 24 hrs. post indomethacin administration and continued for 5 days. The animals in the experimental groups received N-isovaleroyl-L-glutamyl-L-tryptophan or the comparator drug Sucralfate, and the control group received distilled water.

The 30 mg/kg ulcerogenic dose of indomethacin induced ulcers in 100 % of the animals in 24 hrs. After 5 days, the number and size of the lesions in the control group (indomethacin) animals did not change (Table 11).

On the 5^{th} day, only 7 out of 10 animals in the control group survived, while in the group receiving VGT 9 out of 10 survived.

**Table 11**

| ***Effect* of *Treatment with N-isovaleroyl-L-glutamyl-L-tryptophan on Healing of Indomethacin-Induced Ulcers* (M ± *SEM)*** | | | | | | | |
|---|---|---|---|---|---|---|---|
| Groups | # Lesions | Lesion Size | % Animals with Ulcers | PI | Ulcer Index | GA₁ | GA₂ |
| Indomethacin 1 day | 8.4 ± 1.6 | 31.0 ± 7.4 | 100 | 8.4 | 31.0 | | |
| Control Indomethacin 5th day | 8.3 ± 1.0 | 32.6 ± 10.2 | 100 | 8.3 | 32.6 | | |
| Indomethacin +VGT preparation 1 mkg/kg | 4.1 ± 0.8* | 12.3 ± 3.5* | 100 | 4.1 | 12.3 | 2.0 | 2.6 |
| Indomethacin + VGT preparation 10 mkg/kg | 6.2 ± 1.3 | 23.6 ± 6.1 | 100 | 6.2 | 23.6 | 1.4 | 1.4 |
| Indomethacin + Sucralfate 200 mg/kg | 3.3 ± 1.0* | 11.5 ± 4.26* | 85.7 | 2.8 | 9.86 | 3.0 | 3.3 |

| | | | | | | | |
|---|---|---|---|---|---|---|---|
| *- Significant difference compared to control group, P < 0.05 GA₁₋ gastroprotective activity calculated for number of lesions GA₂ - gastroprotective activity calculated for lesion size | | | | | | | |

Treatment with N-isovaleroyl-L-glutamyl-L-tryptophan and also with Sucralfate reduced the number and size of gastric lesions. The therapeutic effect of N-isovaleroyl-L-glutamyl-L-tryptophan was stronger at the 0.1 mkg/kg dose, survival of the animals also went up (9 out of 10 survived). There was a significant reduction in the number and size of the lesions, Pauls index was 2 times lower than that of the control animals, and gastroprotective activity was 2 and 2.6. As to the therapeutic ability to reduce the number and size of the lesions, VGT preparation in a 0.1 mkg/kg dose did not significantly differ from the comparator drug Sucralfate. However, only 85.7 % of the animals in the group receiving Sucralfate were found to have lesions, thus, PI and GA in this group were somewhat higher than those in the group receiving N-isovaleroyl-L-glutamyl-L-tryptophan.

Following the changes in body weights of the animals (Table 12) revealed that the animals administered ulcerogenic doses of indomethacin presented with a 17.6 % reduction of this indicator on day 5 as compared to the intact animals. Some loss of weight of the intact animals could be contributed to the 18-hr fasting. VGT did not cause any significant changes of this indicator as compared to the intact animals.

**Table 12**

| **Effect of *N-isovaleroyl-L-glutamyl-L-tryptophan* on Changes in Body Weight of Animals with Indomethacin Intoxication (M ± SEM)** | | |
|---|---|---|
| Groups | Body weight | |
| | Before Indomethacin | In 5 days |
| Intact Animals | 100 | 93.5 ± 0.9 |
| Control 30 mg/kg Indomethacin | 100 | 82.4 ± 0.9# |
| VGT Preparation 0.1 mkg/kg | 100 | 84.4 ± 2.3# |
| VGT Preparation 10.0 mkg/kg 0 | 100 | 84.1 ± 3.0# |
| Sucralfate 200 mg/kg | 100 | 86.6 ± 2.5 |

| | | |
|---|---|---|
| # - Significant difference compared to intact animals, P < 0.05 | | |

Formation of lesions in gastric mucosa was followed by elevated LP intensity. Indomethacin reduced SODM activity and elevated activity of MDA in the ulcerogenic zone already in 24 hrs., and on the 5^{th} day, SODM activity increased and MDA level was twice as high as the norm (Table 13).

**Table 13**

| ***Therapeutic Effect* of *N-isovaleroyl-L-glutamyt-L-tryptophan* on the *Activity of Superoxide Dismutase and on Malonic Anhydride Content in Blood* Se*rum* of *Rats with Indomethacin Intoxication (M* ± *SEM)*** | | |
|---|---|---|
| Groups | SODM (conv. units/ml) | MDA (OD₅₃₂) |
| Intact Animals | 2.82 ± 0.21 | 0.103 ± 0.016 |
| Control Indomethacin, 24 hrs. | 1.30 ± 0.08# | 0.188 ± 0.011# |
| Control Indomethacin, 5 days | 2.34 ± 0.10 | 0.245 ± 0.017# |
| VGT Preparation 0.1 mkg/kg | 3.1 ± 0.11* | 0.169 ± 0.012*# |
| VGT Preparation 10.0 mkg/kg | 3.06 ± 0.16* | 0.217 ± 0.030# |
| Sucralfate 200 mg/kg | 2.66 ± 0.11 | 0.155 ± 0.037* |

| | | |
|---|---|---|
| # - Significant difference compared to intact animals, P < 0.05 *- Significant difference compared to control group, P < 0.05 | | |

A small dose of VGT reduced MDA content by 70 % and increased SODM activity. Intoxication with indomethacin led to the reduction of ALT activity and elevated AP. Both doses of N-isovaleroyl-L-glutamyl-L-tryptophan boosted reduced ALT activity, albeit not to the level characteristic of the intact animals, and lowered activity of AP. Notably, the greatest impact was observed at the lower dose of the BGT preparation (Table14).

**Table 14**

| ***Effect of Treatment with N-isovaleroyl-L-glutamyl-L-tryptophan on the Activity of Alanine Aminotransferase and Alkaline Phosphatase in Blood Serum of Rats with Indomethacin-Induced Ulcers (M ± SEM)*** | | |
|---|---|---|
| Groups | ALT (mM/h*L) | AP (nM/c*L) |
| Intact Animals | 1.08 ± 0.03 | 1037 ± 65 |
| Control Indomethacin | 0.52 ± 0.09# | 1607 ± 147# |
| VGT Preparation 0.1 mkg/kg | 0.75 ± 0.09# | 1357 ± 58 |
| VGT Preparation 10.0 mkg/kg | 0.66 ± 0.11* | 1368 ± 148 |
| Sucralfate 200 mg/kg | 0.69 ± 0.05# | 1260 ± 104 |

| | | |
|---|---|---|
| # - Significant difference compared to intact animals, P < 0.05 *- Significant difference compared to control group, P < 0.05 | | |

These results also show that the ulcerogenic dose of indomethacin causes adrenal hypertrophy as well as involution of the spleen and thymus. Thymus involution was highly pronounced - the weight of the organ was reduced almost in half (Table 15).

**Table 15**

| **Effect of Treatment with N-isovaleroyl-L-glutamyl-L-tryptophan on the Weight of Organs of Rats with Indomethacin-Induced Lesions (M ± SEM)** | | | | | |
|---|---|---|---|---|---|
| Groups | Wt. of the Animals (g) | Liver | | Spleen | |
| | | Wt., g | Rel. wt., % | Wt., g | Rel. wt., % |
| Intact Animals | 248±5 | 7.468 ± 0.221 | 2.96 ± 0.104 | 1.206 ± 0.072 | 0.48 ± 0.03 |
| Indomethacin, 5 days | 229 ± 7 | 7.509 ± 0.656 | 3.25 ± 0.21 | 1.080± 0.084 | 0.47± 0.03 |
| VGT Preparation 0.1 mkg/kg 0 | 217±9 | 7.062± 0.411 | 3.27 ±0.18 | 1.061 ± 0.085 | 0.48 ± 0.03 |
| VGT Preparation 10.0 mkg/kg | 224 ± 16 | 8.310 ± 0.426 | 3.76 ± 0.020* | 1.233 ± 0.104 | 0.56 ± 0.05 |
| Sucralfate 200 mg/kg | 223 ± 12 | 7.158 ± 0.330 | 3.23 ± 0.13 | 1.260 ± 0.083 | 0.57 ± 0.04 |
| | | | | | |

| Groups | Wt. of the Animals (g) | Adrenal Glands | | Thymus | |
|---|---|---|---|---|---|
| | | Wt., | Rel. wt., % | Wt., g | Rel. wt., % |
| Intact Animals | 248 ± 5 | 41.6 ± 1.4 | 0.017 ± 0.001 | 0.312 ± 0.011 | 0.124 ± 0.006 |
| Indomethacin, 5 days | 229 ± 7 | 55.1 ± 5.1# | 0.024 ± 0.002 | 0.151± 0.013# | 0.066± 0.005# |
| VGT Preparation 0.1 mkg/kg 0 | 217±9 | 41.8 ± 5.1* | 0.019 ± 0.002 | 0.173 ± 0.018# | 0.081 ± 0.007 |
| VGT Preparation 10.0 mkg/kg | 224 ± 16 | 48.3 ± 2.2 | 0.022 ± 0.002 | 0.179 ± 0.032# | 0.078 ± 0.008 |
| Sucralfate 200 mg/kg | 223 ± 12 | 50.1 ± 3.2 | 0.023± 0.002 | 0.155± 0.011# | 0.070 ± 0.005# |

| | | | | | |
|---|---|---|---|---|---|
| # - Significant difference compared to intact animals, P < 0.05 *- Significant difference compared to control group, P < 0.05 | | | | | |

Treating indomethacin intoxication with VGT lowered the elevated weight of the adrenal glands. Table 15 shows that the proposed preparations did not make any significant changes in the weight of the thymus, but there was some tendency toward an increase in the reduction of weight of the thymus when using N-isovaleroyl-L-glutamyl-L-tryptophan.

Thus the conclusion derived from the obtained results is that N-isovaleroyl-L-glutamyl-L-tryptophan exhibits a therapeutic effect on the indomethacin-induced ulcerogenesis model, and the highest therapeutic effect was shown with a 0.1 mkg/kg dose.

These data show that the claimed N-isovaleroyl-L-glutamyl-L-tryptophan compound inhibits the formation of ulcers in gastric mucosa and may be used as a therapeutic and preventive antiulcer agent.

### Industrial Applicability

The claimed preparation can be manufactured at pharmaceutical or chemical plants specializing in organic synthesis.

## Claims

1. N-isovaleroyl-L-glutamyl-L-tryptophan with the general formula: 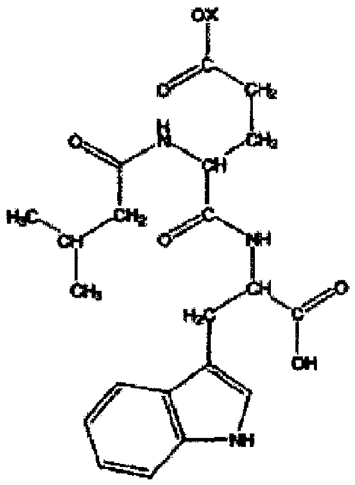 wherein X = H, Na, K, having a molecular weight of 417.5 - 465.5 as is an agent preventing the formation of gastric and duodenal ulcers.

## Patentansprüche

1. N-Isovaleroyl-L-glutamyl-L-tryptophan der allgemeinen Formel 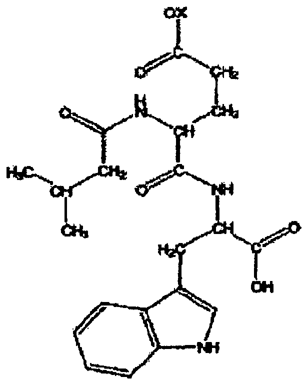 wobei X = H, Na, K mit einem Molekulargewicht von 417,5 - 465,5 als Mittel zur Vorbeugung der Bildung eines Magen- oder Zwölffingerdarmgeschwürs.

## Revendications

1. N-isovaleroyl-L - glutamyl -L- tryptophane avec la formule générale : 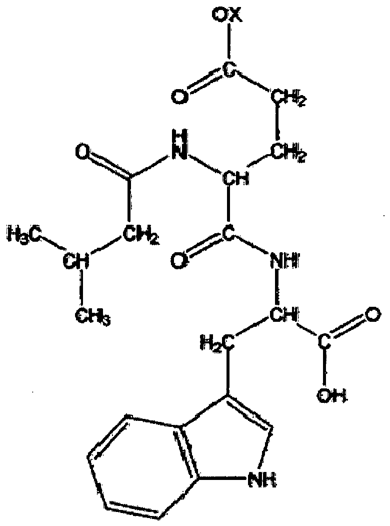 dans laquelle X = H, Na, K, d'un poids moléculaire compris entre 417,5 et 465,5 en tant que les précautions capables d'obvier à la formation d'un ulcère gastro-duodénal.
